# EUROPEAN PATENT APPLICATION

(11) **EP 1 854 435 A2**
(43) Date of publication of application: **14.11.2007**
(21) Application number: 07107422.3
(22) Date of filing: 03.05.2007
(51) Int. Cl.: A61F 5/02

(54) **A modular element system for the manufacturing of ortheses**

(30) Priority: 09.05.2006 IT MI20060904
(71) Applicant: MCH s.r.l., 20037 Paderno Dugnano (MI) (IT); Cortonesi, Rivo, 6929 Gravesano (CH)
(72) Inventor: Cortonesi, Rivo, 6929, GRAVESANO (CH); Marinoni, Marco, I-20021, BARANZATE (MI) (IT)
(74) Representative: Mittler, Enrico

(57) **Abstract**

There is described a modular element system for the manufacturing of ortheses, which comprises a plurality of prismatic modules (100, 300, 400, 600, 700) variously connectable by means of interlocking connection elements (200, 500, 620, 720, 20, 21, 30, 40, 50). The connection elements (200, 500, 620, 720, 20, 21, 30, 40, 50) may be incorporated and integral with the modules (100, 300, 400, 600, 700), or may be physically separate, but each one of these is provided with at least one cylindrical part (201, 501, 502) adapted to be compliantly interlocked and retained in a cylindrical cavity (101, 301, 341, 401, 441, 601, 711) included in one of said modules (100, 300, 400, 600, 700).

## Description

The present invention relates to a modular element system for the manufacturing of ortheses.

Ortheses are devices that are applied to the body to correct defective mechanical functions, without replacing missing anatomical parts. Various applications are related to the correction of skeletal deformities, such as scoliosis, kyphosis, and lordosis, the prevention of problems caused by senile bone fragility due to osteoporosis, and the stabilisation of fractures.

At present, ortheses are made of a thermoformed plastic material, carbon fibre or with mixed solutions of metal and plastic or carbon fibre.

They display drawbacks which may be summarised in the following items:
- the manufacturing thereof requires a plaster cast or the detection by sophisticated measurement techniques of the concerned part of the human body;
- they are completely rigid and nearly entirely envelop the part of the body they are applied to, with obvious effects of constriction and weakening of the muscles, impairment of skin transpiration, excessive generation of heat with the release of abundant perspiration, friction of the skin with possible formation of wounds;
- in the case of prolonged therapeutic treatment, as in idiopathic scoliosis, they may not be adapted to the various phases of body development of a young patient, and need to be replaced or modified at regular intervals;
- the manufacturing, adjustment or modification thereof requires many sessions, generally at a distance of a few weeks.

It is therefore apparent that ortheses imply heavy expenses and negatively affect the life quality of the patients.

For these reasons, it is desirable to obtain improvements in the technique so as to achieve the following aims:
- to manufacture ortheses without the need to have recourse to plaster casts or to sophisticated measurements, by simply directly manufacturing them on the patient and to immediately carrying out the design and adjustment in only one session;
- to manufacture light ortheses, which allow to maintain uncovered the parts of the body that are subjected to the therapeutic treatment;
- to manufacture ortheses provided with the required degrees of freedom in order not to damage the muscles of the patient;
- to manufacture ortheses which apply specific pressures to the various areas of the body by the use of adjustable pressure pneumatic sleeves;
- to manufacture ortheses which adapt to the body development of the patient, by means of rapid remodelling operations, with only the addition of few elements and without the need to manufacture ortheses again ex novo;
- to minimise discomforts for the patients;
- to considerably cut down the costs for patients and for the national health care system.

It is the object of the present invention to achieve the above mentioned benefits by minimising the above-disclosed disadvantages.

According to the invention, such an object is achieved with a modular element system for ortheses characterised in that it comprises a plurality of modules which are variously connectable by means of interlocking connection elements.

The connection elements may be incorporated and integral with the modules, or they may be physically separate, but each of them is provided with at least one cylindrical part adapted to be compliantly interlocked and retained in a suitable cavity included in one of said modules.

Straight and curved rods, which may be modelled in the three dimensions and are easily adaptable to the human body to manufacture an ortheses made to measure for the patient, may be obtained with consecutive series of compliant interlockings between modules and connection elements in a practical and rapid manner.

These and other features of the present invention will become more apparent from the detailed description of the examples of embodiment depicted by no way of limitation in the accompanying drawing, in which:
figure 1 shows a perspective view of an orthesis for scoliosis made according to the present invention;
figure 2 shows a perspective view of a base module which may be used for the orthesis in figure 1;
figure 3 shows a plan view of the module in figure 2;
figure 4 shows a section view along line IV-IV in figure 3;
figure 5 shows a section view along line V-V in figure 3;
figure 6 shows a section view along line VI-VI in figure 3;
figure 7 shows a section view along line VII-VII in figure 3;
figure 8 shows a section view along line VIII-VIII in figure 3;
figure 9 shows a section view along line IX-IX in figure 4;
figure 10 shows a perspective view of a connection element which may be used for the orthesis in figure 1;
figure 11 shows a perspective view of a connection between modules such as that in figure 2 by means of connection elements such as that in figure 10;
figure 12 shows a perspective view of another connection element which may be used for the orthesis in figure 1;
figure 13 shows a plan view of the module in figure 12;
figure 14 shows a section view along line XIV-XIV in figure 13;
figure 15 shows a section view along line XV-XV in figure 13;
figure 16 shows a section view along line XVI-XVI in figure 13;
figure 17 shows a perspective view of a connection between modules such as that in figure 2 and a module such as that in figure 12 by means of connection elements such as that in figure 10;
figure 18 shows a perspective view of another connection element which may be used for the orthesis in figure 1;
figure 19 shows a perspective view of a connection between modules such as that in figure 2 and a module such as that in figure 18 by means of connection elements;
figure 20 shows a perspective view of another connection element which may be used for the orthesis in figure 1;
figure 21 shows a perspective view of a connection between modules such as that in figure 2 by means of a connection element such as that in figure 20;
figure 22 shows a perspective view of another module with an incorporated connection element which may be used for the orthesis in figure 1;
figure 23 shows a perspective view of another module with a connection element incorporated in a rotating manner which may be used for the orthesis in figure 1;
figure 24 shows a perspective view of a pneumatic sleeve which may be used to cover parts of an orthesis according to the invention;
figure 25 shows a perspective view of the use of the pneumatic sleeve in figure 24;
figure 26 shows a perspective view of an anti-wound sleeve which may be used to cover parts of the orthesis;
figure 27 shows a perspective view of the use of the sleeve in figure 24;
figures 28-40 show other alternatives of possible connections between modules.

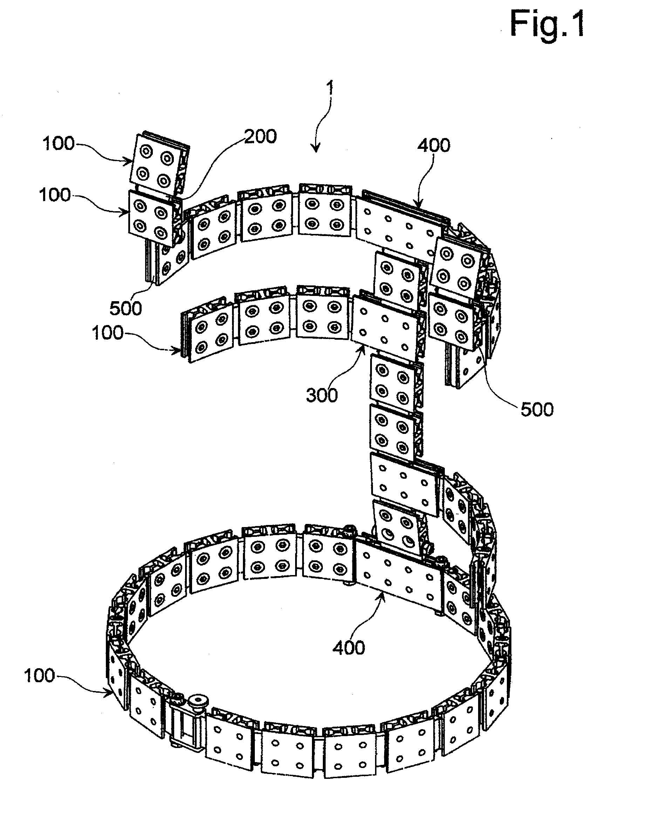

Figure 1 shows an example of an orthesis for scoliosis which employs a plurality of modules 100, 300 and 400 variously connected together by means of connection elements 200 and 500.

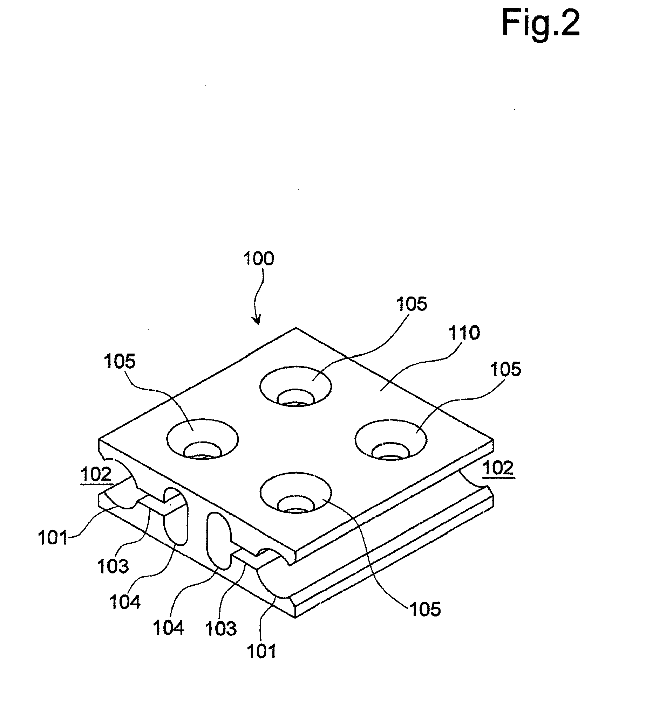

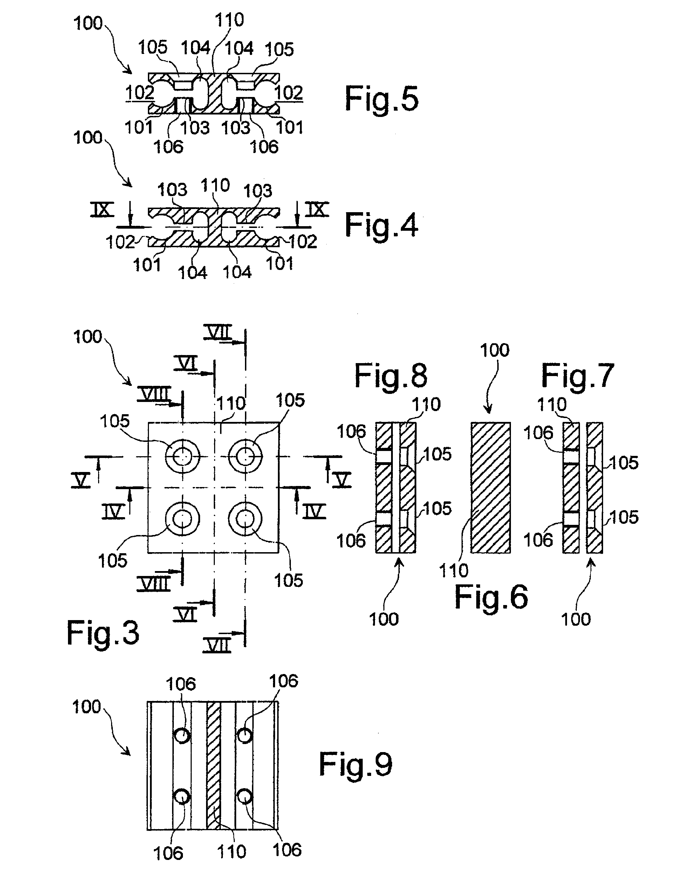

Figures 2-9 depict an example of module 100 formed by a prismatic square body 110, comprising (figures 2, 4 and 5) two cylindrical side cavities 101, each of which communicates with a side opening 102. Furthermore, each cylindrical cavity 101 communicates, through an aperture 103, with an internal cavity 104. Pairs of through holes 105 and 106 communicate with one another through the apertures 103 (figures 2, 3, 5, 7, 8 and 9). Each through hole 105 displays a funnel-shaped flaring, suitable to insert the head of a screw; each through hole 106 is threaded.

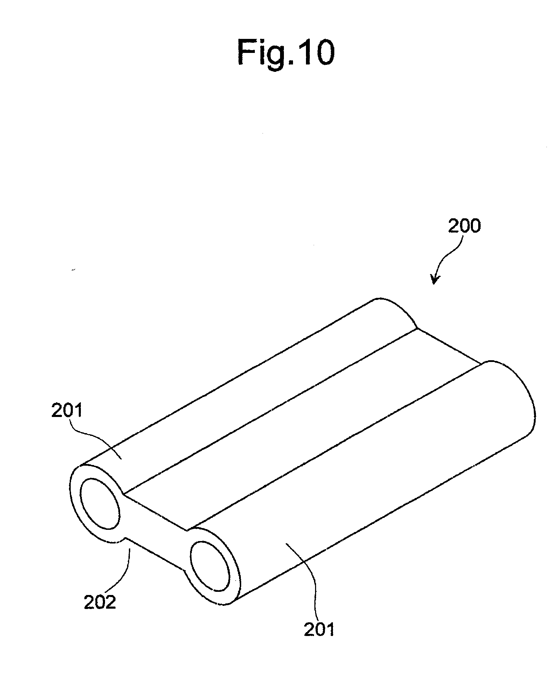

Figure 10 depicts a connection element 200, which is physically separate from module 100. The connection element 200 is formed by two cylindrical side parts 201 and by a central body 202.

The connection element 200 may be used to connect two modules 100 together in an angularly and transversally variable position.

One of the two side parts 201 is inserted within a cylindrical cavity 101. Such an operation is possible because the distance between the walls of the side opening 102 of the module 100 is compatible with the height of the central body 202 of the connection element 200.

Once the above said side part 201 has been inserted within the cylindrical cavity 101, the connection element 200 may still be rotated or shifted: once it has been appropriately positioned, screws may be inserted in the through holes 105, 106, exerting forces which tend to draw the walls of the apertures 103, the walls of the cylindrical cavities 101 and the walls of the side openings 102 together; accordingly, the cylindrical side part 201 of the connection element 200 is held in the cylindrical cavity 101 and retained in the determined position.

The other cylindrical side part 201 of the same connection element 200 is similarly held in a cylindrical cavity 101 of another module 100.

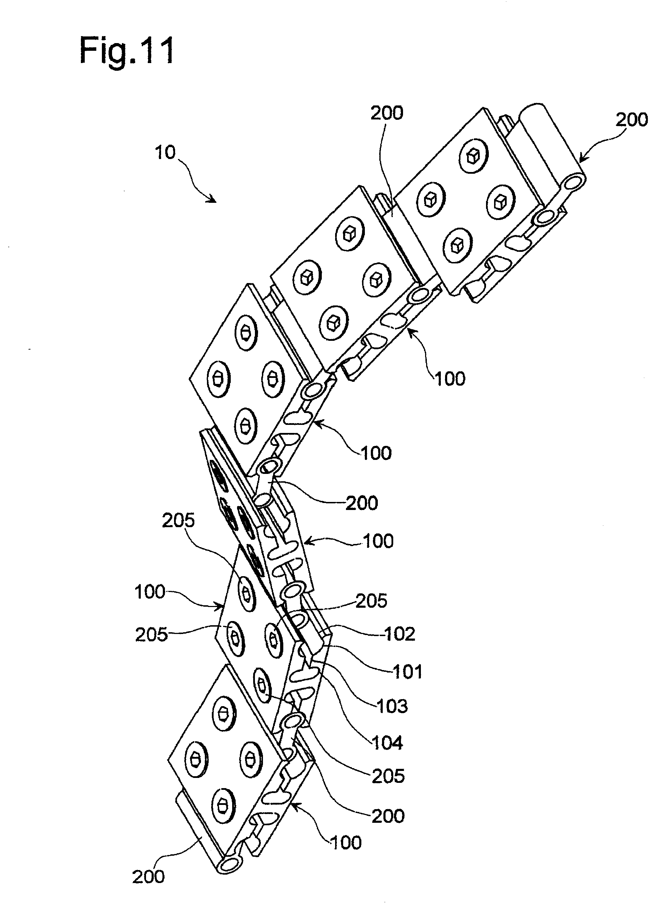

By repeating this procedure with other modules identical to module 100 and other connection elements identical to connection element 200, and alternating at will aligned coplanar arrangements, transversally staggered coplanar arrangements and angled arrangements, it is possible to obtain a structure such as that shown in figure 11, where it is indicated as a whole by numeral 10. Screws 205 inserted in the through holes 105 and 106 may be noted.

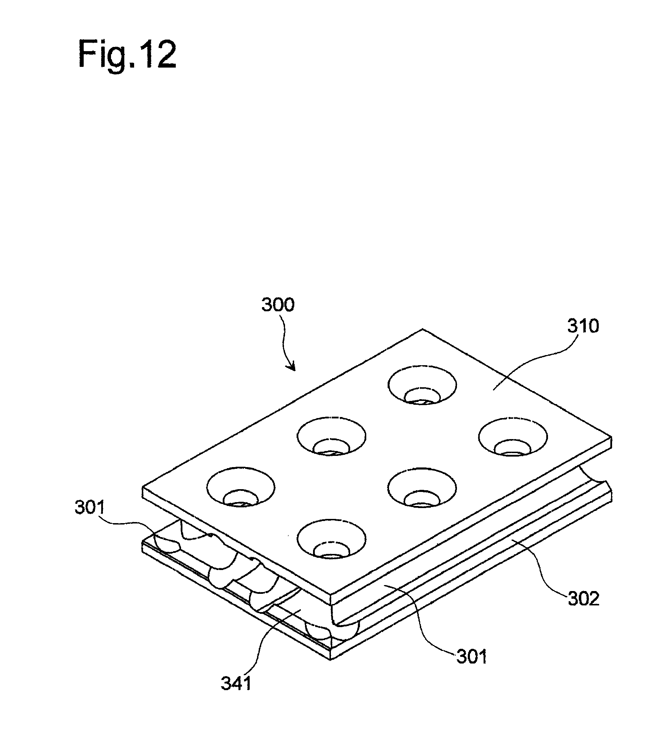

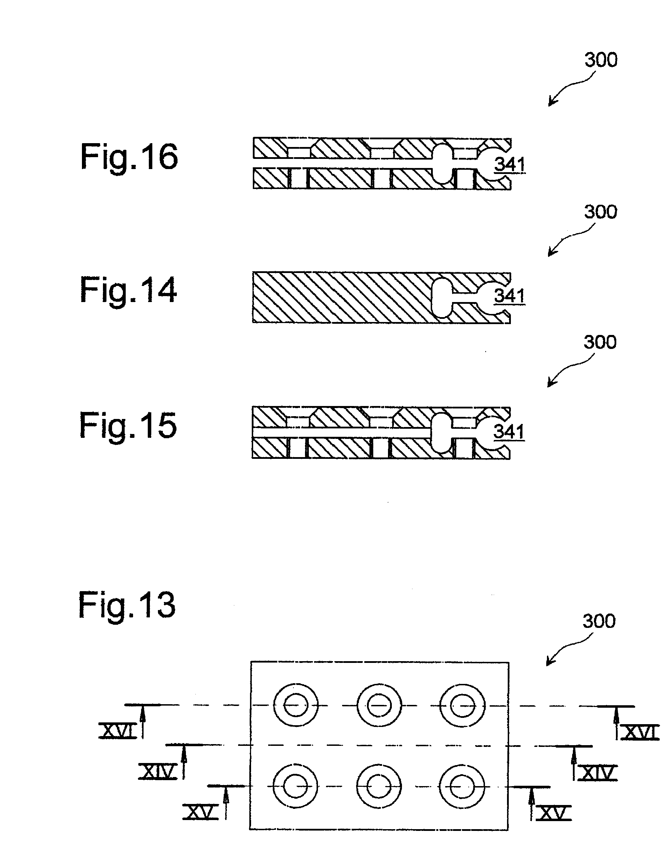

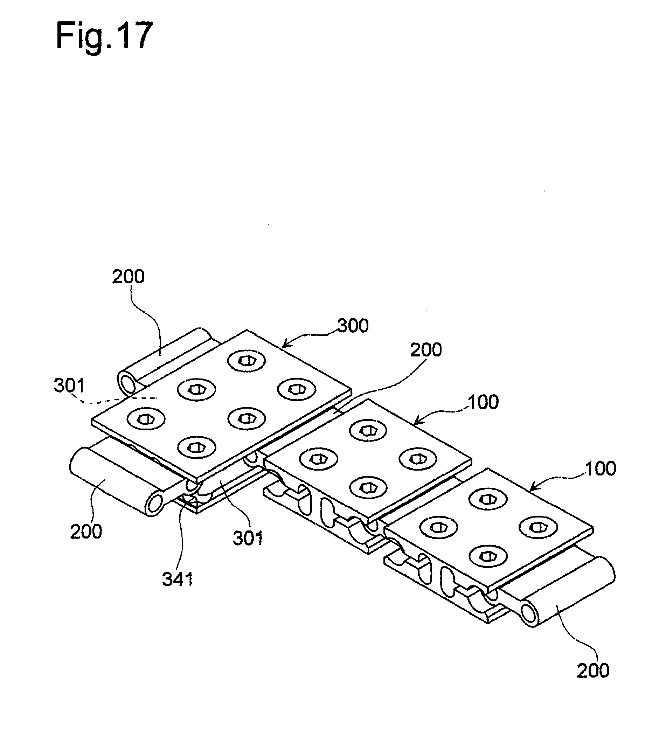

Figures 12-16 depict a second example of module 300. It may be noted that module 300 differs from module 100 because it has a rectangular body 310, which also displays a cylindrical cavity 341 placed on the end of the module 300, as well as two cylindrical side cavities 301 which communicate with the outside through an opening 302. Each of cavities 301 and 341 allows the compliant interlocking with a cylindrical side part 201 of a connection element 200. Figure 17 shows an example of interlocking of the module 300 with a module 100 by means of the interpositioning of a connection element 200.

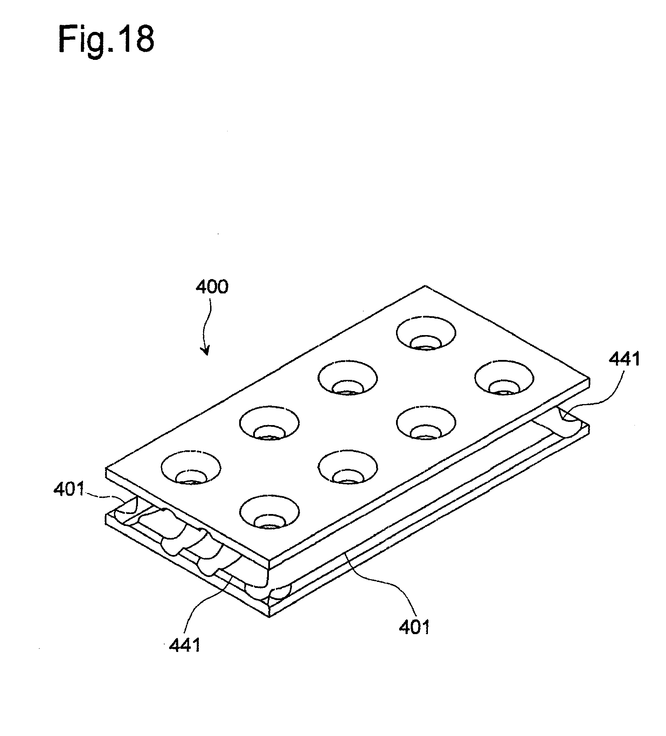

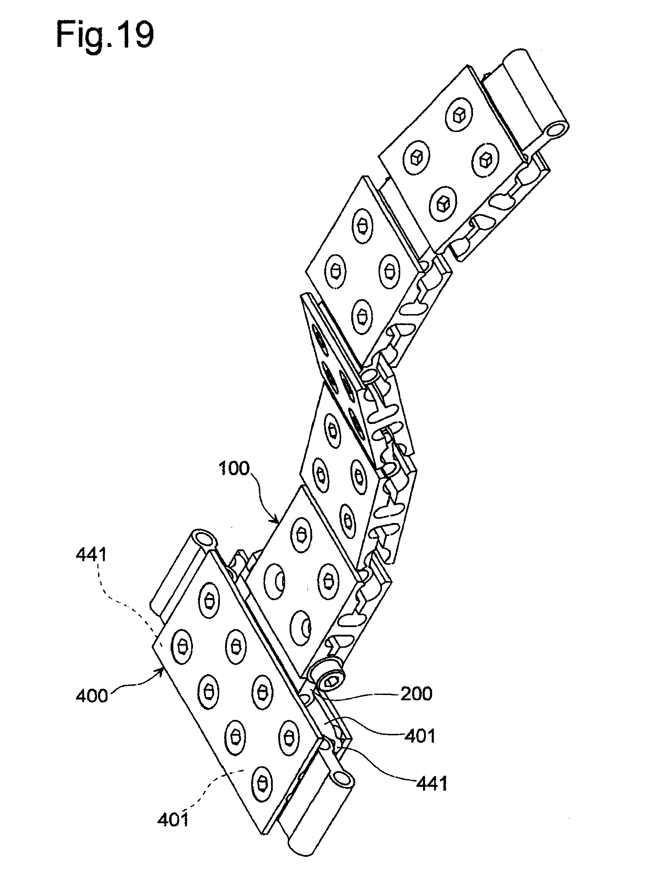

A module 400 displaying two cylindrical side cavities 401 and two cylindrical cavities 441 at the ends is shown in figure 18. This module allows the interlocking of four connection elements 200. Figure 19 shows the way the module 400 interconnects to the module 100 by means of the connection element 200.

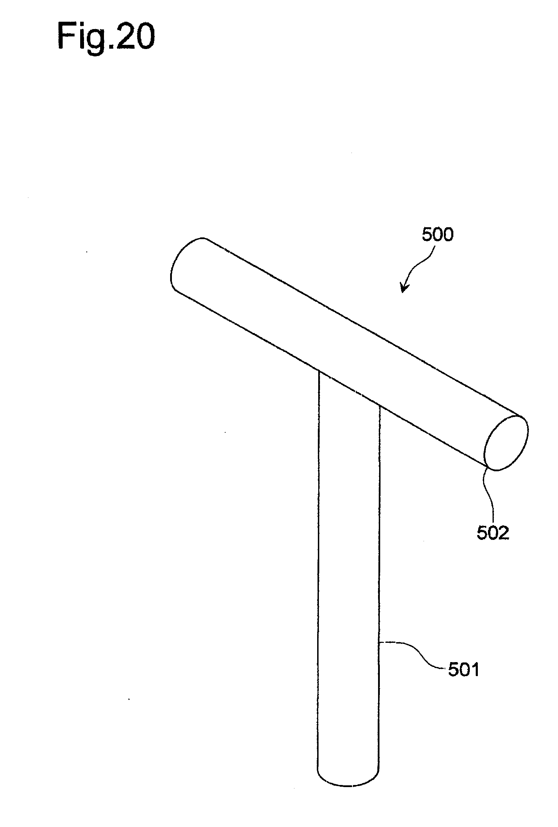

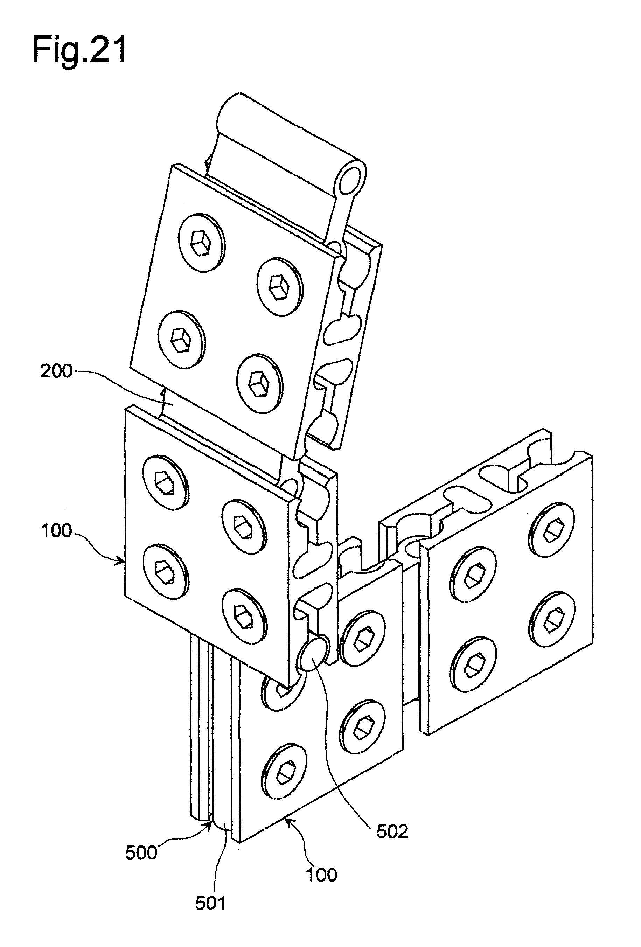

A connection element 500 different from the element 200 is shown in figure 20. It is formed by a cylindrical part 501, having a face adhering and orthogonal to another cylindrical part 502. As both cylindrical parts 501, 502 are suitable to be compliantly interlocked in the previously described cylindrical cavities 101, 301, 341, 401 and 441, two modules 100 may be connected such as in figure 21, or modules 300 or 400, in a similar manner.

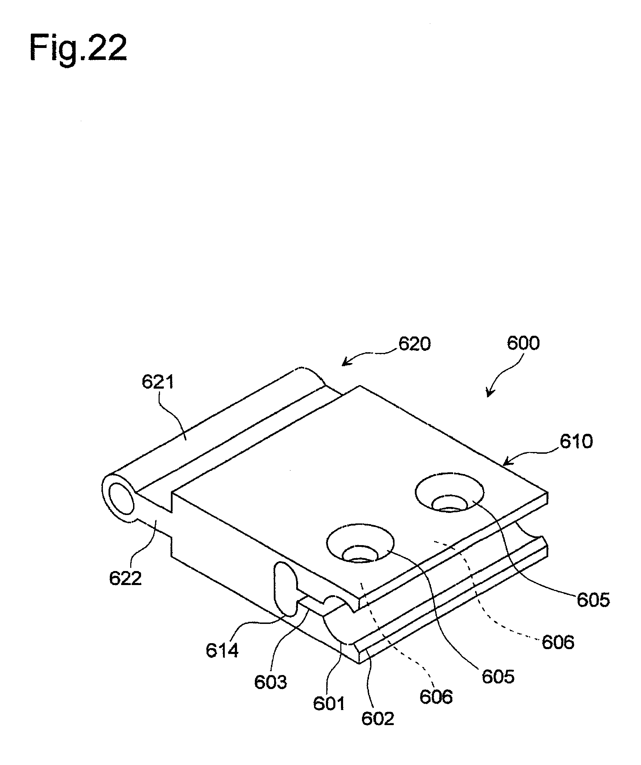

Figure 22 shows a module 600, which may be subdivided in two portions 610 and 620.

The portion 610 is approximately equivalent to half of the module 100 and carries out the same functions thereof. More precisely, the portion 610 comprises a cylindrical cavity 601 communicating with a side opening 602, and, through an aperture 603, with two flared through holes 605 and with two threaded through holes 606. The portion 610 behaves similarly to the modules 100, 300 and 400: the cylindrical cavity 601 allows the compliant interlocking of one of the previously described cylindrical parts 201, 501 or 502.

The second portion 620 is approximately equivalent to half of the element 200 and carries out the same functions thereof it consists of an incorporated connection element comprising a body 622 integral with a cylindrical part 621, insertable in a cylindrical cavity 101, 301, 341, 401, 441 or 601.

It is obvious that the connection between the module 600 and another module 100, 300, 400 or 600 does not require the connection element 200, allowing a more limited use of pieces, even if in a less versatile manner.

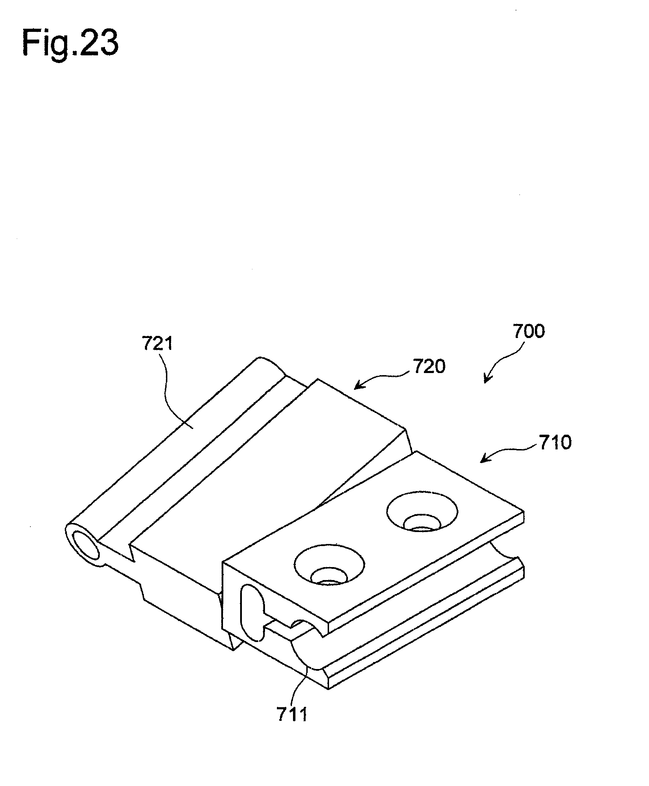

Figure 23 shows another example of module 700 subdivided in two parts connected through mechanisms, which are not shown, which allow the relative rotation, giving a part of the orthesis 1 a degree of freedom, with undoubted benefits for the comfort of the patient. The module 700 may be subdivided in two portions 710, 720.

The first portion 710 comprises a cylindrical cavity 711 which may be used as interlocking means for other connecting elements 200, 500 and 620. The second part 720 incorporates a connection element 720 comprising a cylindrical part 721, which allows the interlocking of cylindrical cavities 101, 301, 341, 401, 441, 601 or 711.

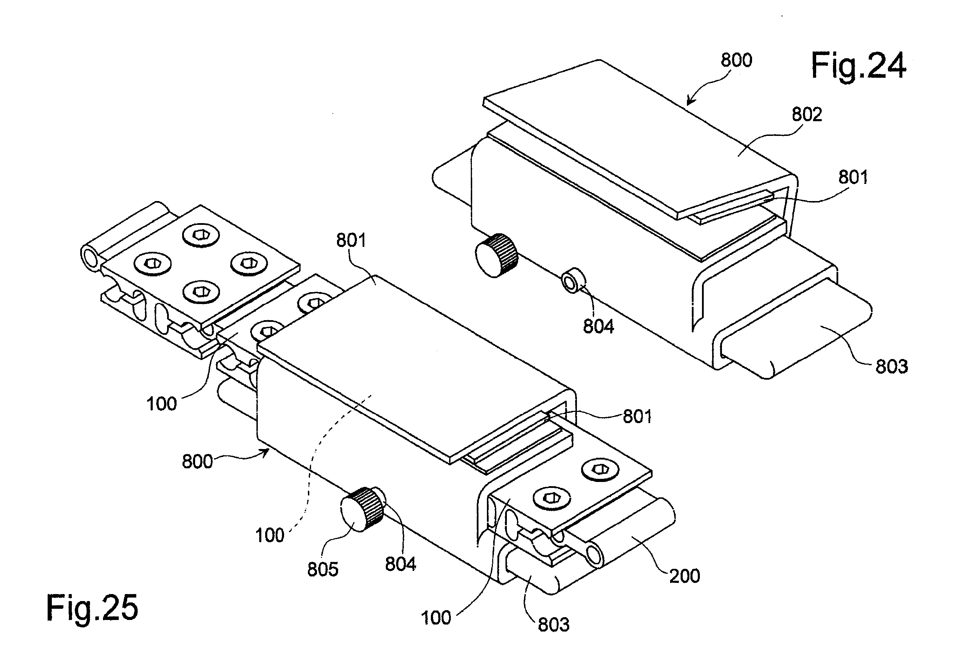

Figure 24 shows a covering 800 for a part of the orthesis 1 with a pneumatic sleeve. There may be noted a layer of velcro 801 covered by biocompatible fabric 802, which also covers an inflatable pad 803 connectable to a pump by means of a small valve 804, shown in an open position.

Figure 25 shows the way the covering 800 is placed around a part of the orthesis 1. The cap 805 closing the valve 804 may be noted.

In this manner, an orthesis 1 may be obtained which is capable of operating in a differential manner depending on the different areas of the body, exerting different pressures in different areas.

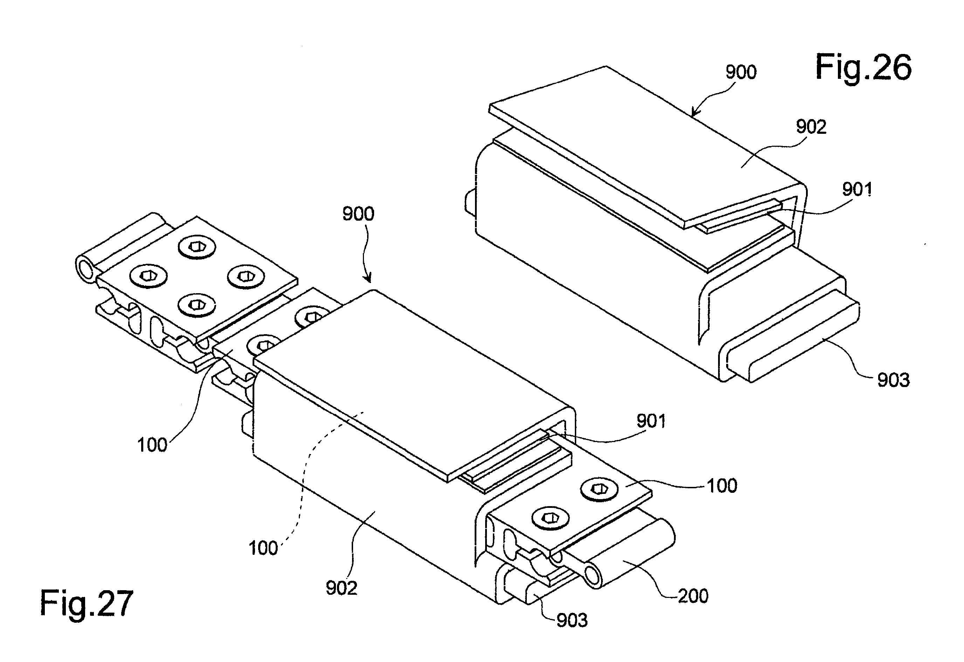

Figure 26 shows a covering 900 with an anti-wound sleeve, it also comprising a layer of velcro 901 covered by biocompatible fabric 802, which also covers a layer of soft material 903.

Figure 27 shows the way the covering 900 is placed around a part of the orthesis 1.

The procedure for the assembly of the orthesis 1 may therefore be obtained in two steps:
- a first step of assembling the above said modules 100, 300, 400, 600, 700 by means of the above said connection elements 200 and 500;
- a second step of covering the modules, for instance with anti-wound sleeves (900) or pneumatic sleeves (800). For every single interconnection of a module 100, 300, 400, 600 or 700 with a connection element 200 or 500, the above said step of assembling consists in three steps:

- a first step of inserting the cylindrical part 201, 501, 502, 621 or 721 in the cylindrical cavity 101, 301, 341, 401, 441, 601 or 711;
- a second step of positioning the connection element with respect to the module by means of reciprocal rotations or shifts;
- a third step of retaining the cylindrical part 201, 501, 502, 621 or 721 within the cylindrical cavity 101, 301, 341, 401, 441, 601 or 711 by means of screws 205.

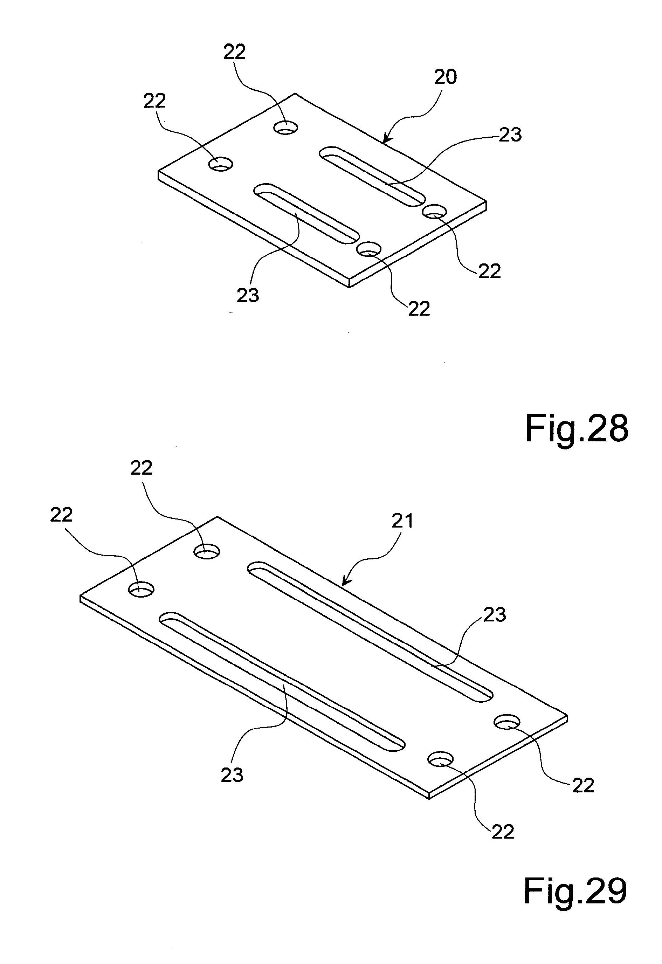

Figure 28 shows other possible alternatives for the connection between modules.

More specifically, figures 28 and 29 show two rectangular plates 20 and 21 having different length, each of which comprises two pairs of holes 22 near the shorter sides of the plates and a pair of elongated slots 23 arranged in an intermediate position, which is asymmetrical in the plate 20 and symmetrical in the plate 21, between the two pairs of holes 22.

One or the other of the two plates 20 and 21 may be used to connect two modules together at a variable distance, such as those indicated by 100, 300, 400, 600 and 700 in the figures 2, 18, 22 and 23.

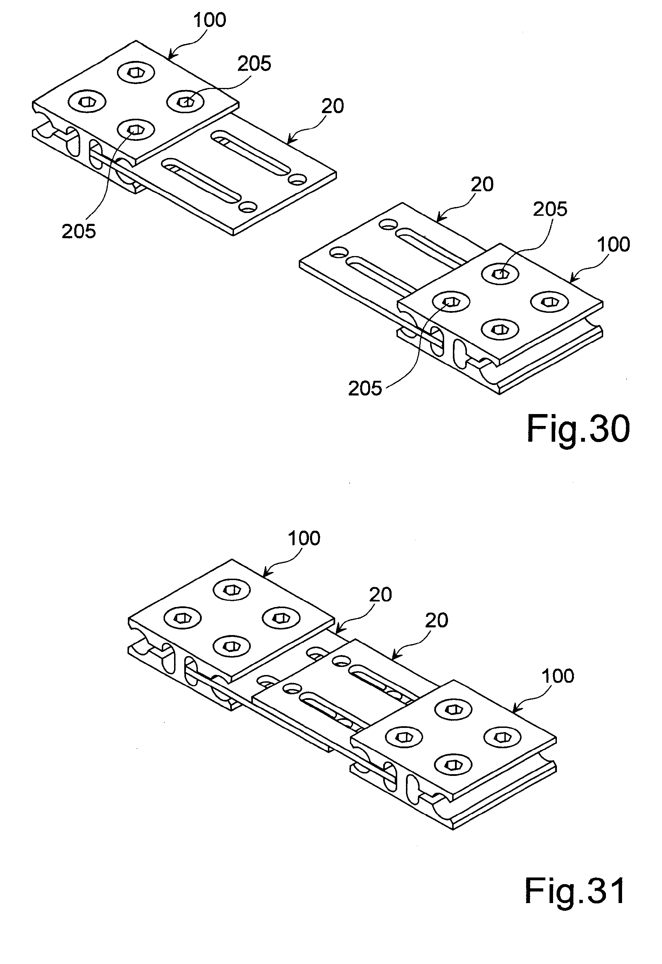

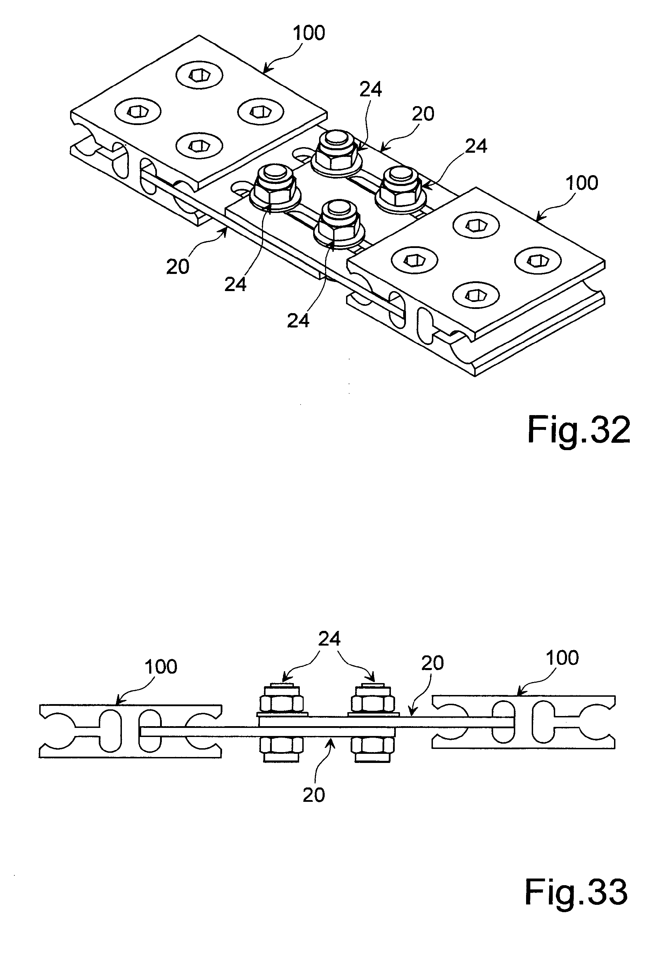

By way of example, figure 30 shows a perspective view of two modules 100, to each of which a plate 20 has been coupled and attached by means of screws 205 inserted in one of the pairs of holes 22. Figure 31 shows the two modules 100 drawn together with the corresponding plates 20 partially overlapped to one another. Figure 32 shows the two plates 20 attached together, along with the corresponding modules 100, by means of self-locking nut-bolt pairs 24 inserted in the slots 23. Figure 33 shows a side view of the same situation as in figure 32.

Obviously, the reciprocal attachment of the plates 20, and therefore of the modules 100, may be rigid or loose depending on whether the auto-locking nut-bolt pairs 25 are well tightened or designed so as to allow a certain remaining clearance. In the first case the nut-bolt pairs serve as an adjustment register for the distance between the modules 100. In the second case the two modules 100 are allowed a limited possibility of motion so as to follow the movements of the person wearing the orthesis.

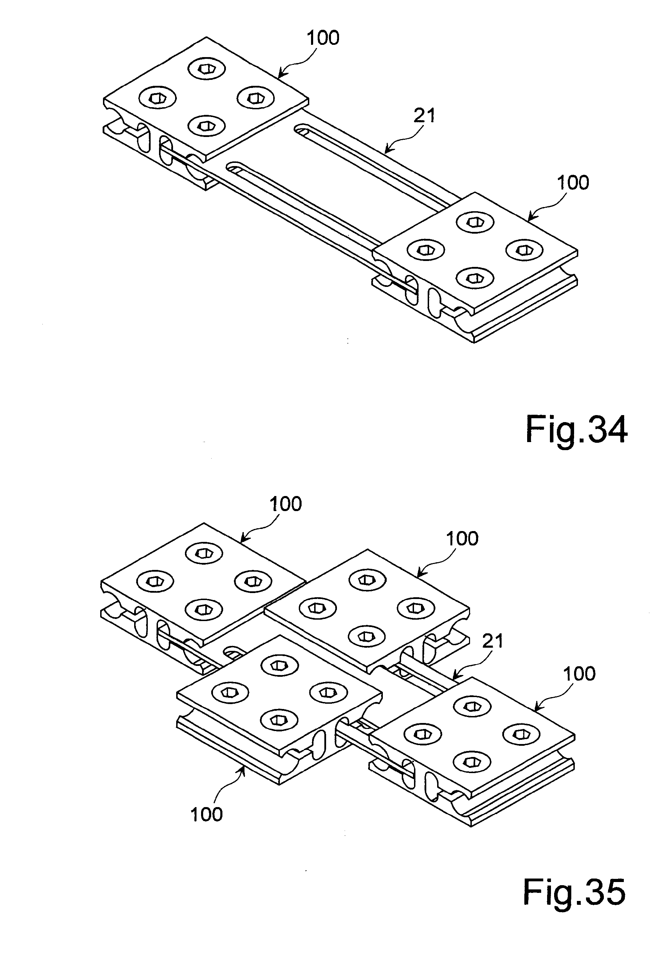

Figures 34 and 35 show, instead, examples of connections between modules 100 which may be obtained with a plate 21.

It must be noted that the plates 20 and 21 may be made of rigid material, as may all other components which have been previously described, or of flexible material, for example of harmonic steel. In the latter case the plates 20 and 21 may bend in a natural manner to follow the movements of the person.

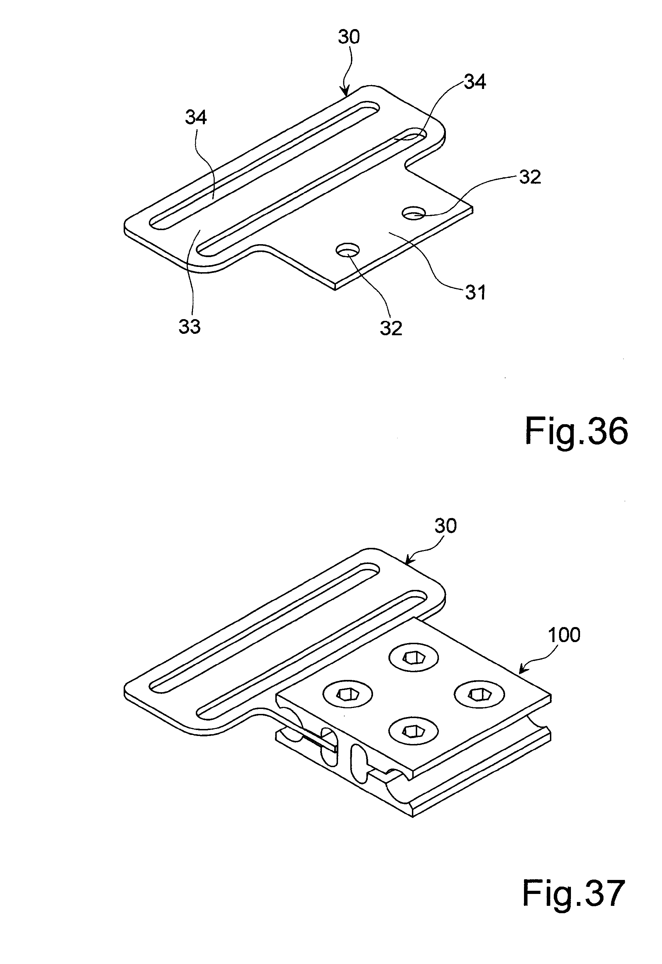

Figure 36 shows a different type of plate 30, which has a first rectangular clear space 31 provided with a pair of holes 32 and a second elongated clear space 33 having rounded corners, in turn provided with elongated slots 34.

Figure 37 shows a plate 30 attached to a module 100 for the connection thereof to other modules, possibly arranged in a staggered position by using the side extension of the slots 34.

The plate 30 may also be made either of rigid material or of flexible material.

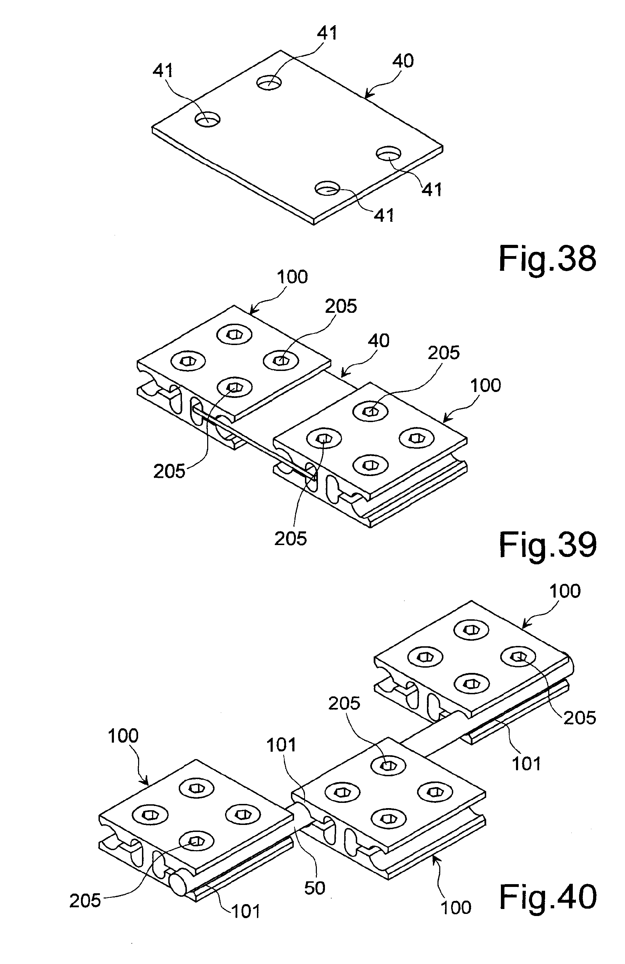

Figure 38 shows a rectangular plate 41, which may be made either of rigid material or of flexible material and comprises two pairs of holes 41.

Figure 39 shows a plate 40 used for the reciprocal connection of two modules 100, the reciprocal distance of which is determined by the distance between the two pairs of holes 41, in which locking screws are inserted 205.

Finally, figure 40 shows three modules 100 connected together in a staggered, possibly also rotated, position by means of a connection element consisting of a rigid cylindrical bar.

The locking may be rigid or may rotate depending on the degree of tightness of the screws 205 of the modules 100.

## Claims

1. A modular element system for the manufacturing of ortheses (1) **characterised in that** it comprises a plurality of prismatic modules (100, 300, 400, 600, 700) variously connectable by means of interlocking connection elements (200, 500, 620, 720, 20, 21, 30, 40, 50).

2. A system according to claim 1, **characterised in that** at least one (100, 300, 400) of said modules (100, 300, 400, 600, 700) is formed by a prismatic body (110, 310, 410) displaying at least one cylindrical side cavity (101, 301, 401) communicating with the side opening (102, 302, 402).

3. A system according to claim 2, **characterised in that** said cylindrical cavity (101, 301, 401) communicates, through apertures (103), with through holes (105, 106) suitable to accommodate threaded means (205) adapted to draw the walls of said apertures (103), said cylindrical cavity (101, 301, 401) and said side opening (102, 302, 402) together, so as to hold, retain and prevent the motion of an external cylindrical body (201) interlocked within said cylindrical cavity (101, 301, 401).

4. A system according to any of claims 2-3, **characterised in that** said prismatic body (110) is square and provided with two of said side cavities (101).

5. A system according to any of claims 2-3, **characterised in that** said prismatic body (310, 410) is rectangular with two of said side cavities (301, 401) and at least one cylindrical end cavity (341, 441).

6. A system according to any of claims 2-5, **characterised in that** at least one (600, 700) of said modules (100, 300, 400, 600, 700) incorporates a connection element (620, 720) comprising a cylindrical part (621, 721) adapted to be compliantly interlocked and retained in one of the cylindrical cavities (101, 301, 341, 401, 441, 601, 711) of an adjacent module (100, 300, 400, 600, 700).

7. A system according to claim 6, **characterised in that** said incorporated connection element (620) is integral with said module (600).

8. A system according to claim 6, **characterised in that** said incorporated connection element (720) is rotatably connected to the remaining portion of said module (700).

9. A system according to any of claims 2-9, **characterised in that** at least one (200, 500, 620, 720, 50) of said connection elements (200, 500, 620, 720, 20, 21, 30, 40, 50) is provided with a cylindrical part (201, 501, 502) adapted to be compliantly interlocked and retained in one of the cylindrical cavities (101, 301, 341, 401, 441, 601, 711) included in one of said modules (100, 300, 400, 600, 700).

10. A system according to claim 9, **characterised in that** at least one (200) of said connection elements (200, 500, 620, 720, 20, 21, 30, 40, 50) incorporates two parallel cylindrical parts (201), each of which (201) is insertable in one of the cylindrical cavities (101, 301, 341, 401, 441, 601, 711) included in one of said modules (100, 300, 400, 600, 700).

11. A system according to claim 9, **characterised in that** at least one (500) of said connection elements (200, 500, 620, 720, 20, 21, 30, 40, 50) consists of two orthogonal cylindrical parts (501, 502), each of which is insertable in one of the cylindrical cavities (101, 301, 341, 401, 441, 601, 711) included in one of said modules (100, 300, 400, 600, 700).

12. A system according to any of claims 1-11, **characterised in that** at least one (20, 21) of said connection elements (200, 500, 620, 720, 20, 21, 30, 40, 50) consists of a rectangular plate (20, 21) which comprises two pairs of holes (22) near the shorter sides of the plate and a pair of elongated slots (23) arranged in an intermediate position between the two pairs of holes (22).

13. A system according to any of claims 1-12, **characterised in that** at least one (30) of said connection elements (200, 500, 620, 720, 20, 21, 30, 40, 50) consists of a plate (30) which has a first rectangular clear space (31) provided with a pair of holes (32) and a second elongated clear space (33) having rounded corners, in turn provided with elongated slots (34).

14. A system according to any of claims 1-13, **characterised in that** at least one (40) of said connection elements (200, 500, 620, 720, 20, 21, 30, 40, 50) consists of a plate (40) provided with two pairs of holes (41).

15. A system according to any of claims 12-14, **characterised in that** said plate (20, 21, 30, 40) is made of flexible material.

16. A system according to any of claims 12-14, **characterized in that** said plate (20,21,30,40) is made of rigid material.

17. A system according to any of the preceding claims, **characterised in that** it comprises a covering element (800) consisting of an adjustable pressure pneumatic sleeve (800).

18. A system according to any of the preceding claims, **characterised in that** it comprises a covering element (900) comprising an anti-wound sleeve (900).

19. A system according to any of claims 17-18, **characterised in that** said covering element (800, 900) comprises a layer of velcro (801, 901) covered by biocompatible material (802, 902).
